(19) Europäisches Patentamt
European Patent Office
Office européen des brevets

(11) Publication number: 0 219 580 B1

(12) # EUROPEAN PATENT SPECIFICATION

(45) Date of publication of the patent specification: 29.11.89

(51) Int. Cl.⁴: C07D 307/935, C07F 7/08

(21) Application number: 85306640.5

(22) Date of filing: 18.09.85

(54) **Stereo-selective reduction.**

(43) Date of publication of application: 29.04.87 Bulletin 87/18

(45) Publication of the grant of the patent: 29.11.89 Bulletin 89/48

(84) Designated Contracting States: BE CH DE FR GB IT LI NL SE

(56) References cited:
EP-A- 0 024 943

JOURNAL OF THE AMERICAN CHEMICAL SOCIETY, vol. 103, no. 18, 9th September 1981, pages 5454-5459, American Chemical Society, Columbus, Ohio, US; A.L. GEMAL et al.: "Lanthanoids in organic synthesis. 6. The reduction of alpha-enones by sodium borohydride in the presence of lanthanoid chlorides: synthetic and mechanistic aspects" 000

(73) Proprietor: THE UPJOHN COMPANY, 301 Henrietta Street, Kalamazoo, Michigan 49001(US)

(72) Inventor: Pearlman, Bruce Allen, c/o The Upjohn Company 301 Henrietta Street, Kalamazoo Michigan 49001(US)

(74) Representative: Perry, Robert Edward et al, GILL JENNINGS & EVERY 53-64 Chancery Lane, London WC2A 1HN(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to a stereoselective reduction process for the preparation of bicyclic lactones which are useful as precursors of prostaglandins. The prostaglandins are known to be useful for a wide variety of pharmaceutical purposes.

Initially, prostaglandins were isolated from natural materials. However, several routes for the total chemical synthesis of the prostaglandins are now known, e.g. as described by Corey et al, in J.A.C.S. 91 (1969) 5675 and J.A.C.S. 92 (1970), and as disclosed in US-A 3 711 515. These and many other synthetic routes proceed via a bicyclic lactone intermediate such as that of formula I.

Most of the commercially important prostaglandins are of the "natural" configuration, i.e. the same stereochemical configuration as the prostaglandins produced in the mammalian metabolism. In particular, the stereochemical configuration of the hydroxy group at the 15-position of many of the commercially-important prostaglandins is natural (i.e. alpha).

Two such commercially-important prostaglandins are $PG_{2\alpha}$ (see US-A 3 706 789) which is marketed by The Upjohn Company for estrous synchronisation, and 11-deoxy-11,16,16-trimethyl-$PGE_2$ (see US-A 4 052 446), a proposed anti-ulcer drug of Hoffmann-La Roche, Inc.

In certain synthetic schemes for the preparation of prostaglandins, intermediates such as that of formula I are prepared from the corresponding ketones such as that of formula II. While numerous means exist for the reduction of this ketone, many are not stereospecific, i.e. they result in a mixture of epimers wherein the hydroxy group is in the alpha or beta position.

A number of reagents stated to be effective for stereoselective reduction of the 15-position of prostaglandins have been described. Thus, diisobutylaluminium 2,6-di-t-butyl-4-methylphenoxide (DIBAL-BHT) has been described by Iguchi et al, Bull. Chem. Soc. Japan, 54:3033 (1981) and J. Org. Chem., 44:1363 (1979). Certain lithium aluminium hydride compounds have been disclosed for this purpose in US-A 4 284 581 and by Noyori et al, J.A.C.S. 101:5843 (1979) and Pure, Appl. Chem. 53:2315 (1981). Lithium thexyl limonyl borohydride (formed by treatment of limonene with thexyl borane and t-BuLi) is utilised by Corey et al, J.A.C.S. 94:8616 (1972). Lithium methyldiisopinocamphenyl borohydride and lithium-t-butyl-diisopinocamphenyl borohydride are employed by Corey et al, J.A.C.S. 93:1491 (1971). DE-A 2 257 162 discloses the use of sodium cyanodiisopinocamphenyl borohydride. US-A 4 247 635 discloses the use of certain microbes for this reduction. Hutton et al, Syn. Com. 9:799 (1979), disclose the use of various reagents produced by treatment of 3–0-benzyl-monocyclohexanone glucose with 1.0 equiv. lithium aluminium hydride and 1.0 equiv. of a monohydroxylic compound such as ethanol, adamantyl alcohol or adamantylmethyl alcohol. GB-A 1 498 764 discloses the use of potassium trialkyl borohydrides.

However, while each of these reagents is effective for stereospecific reduction of particular prostaglandin intermediates, none is effective for all such compounds.

Moreover, many of these reagents are optically active, which means that their use is limited to optically active prostanoid enone substrates. Further, none of these reagents is effective for the stereoselective reduction of a prostaglandin intermediate such as that of formula III. In addition, the reagents noted above are quite expensive, and produce product mixtures from which the product can be isolated only with some difficulty. The only exception is DIBAL/BHT which, as noted below, is not effective for stereoselective reduction of the compound of formula III.

The use of sodium borohydride in the presence of cerium trichloride trihydrate, as a reducing agent, is disclosed by Luche, J.A.C.S. 100:2226 (1978), and Gemal et al, J.A.C.S. 103:5454 (1981). However, these references do not describe a stereoselective reduction of prostaglandins. US-A 4 301 164 (column 25) discloses the use of cerium trichloride and sodium borohydride for the reduction of certain prostanoid C-15 ketones that are susceptible to 13,14-double bond saturation with conventional reagents such $Zn(BH_4)_2$. However, this specification does not describe a stereoselective reduction ($\alpha/\beta$ is 43/57: See Example 97).

A process according to the present invention, for the stereospecific separation of a compound of the formula A-2, comprises reducing a compound of formula A-1 with a borohydride salt in the presence of a trivalent lanthanide salt. Formulae A-1 and A-2, and the reaction sequence, are shown in Chart A. In these formulae: $R_1$ is methyl or $OR_{18}$, $R_{18}$ being a silyl protecting group;

$R_2$ is $-O-(PhX)$, $-C_pH_{2p}-(PhX)$, $-C_mH_{2m}-(DZ)$, $-C_pH_{2p+1}$, $-CH_2-CH_2-CH=C(CH_3)_2$, 3-thienyloxy or $-C_pH_{2p}-(Py)$; and

$L_1$ is $\alpha$–$R_9$:$\beta$–$R_{10}$, $\alpha$–$R_{10}$:$\beta$–$R_9$, $\alpha$–$OR_8$:$\beta$–$R_7$ or $\alpha$–$R_7$:$\beta$–$OR_8$;

in which (PhX) is phenyl optionally substituted by up to 3 substituents independently selected from $C_{1-4}$ alkyl, Cl, F, Br, $NO_2$, $CF_3$, OH and $C_{1-4}$ alkoxy;

(DZ) is a $C_{3-6}$ cycloaliphatic radical optionally substituted by up to 3 substituents as defined above for (PhX);

(Py) is 2, 3 or 4-pyridinyl;

$R_7$ and $R_8$ are independently selected from H and $C_{1-4}$ alkyl;

$R_9$ and $R_{10}$ are independently selected from H, $C_{1-4}$ alkyl and F;

m is zero, 1, 2 or 3; and

p is zero or an integer of from 1 to 8.

Compounds of formula A-1 may exist in optically pure form or as a racemic mixture. Both are included in the scope of this invention.

When $R_1$ is methyl, $R_2$ is n–$C_4H_9$, and $L_1$ is α–$CH_3$:β–$CH_3$, the process is used to prepare 11-deoxy-11,16,16-trimethyl-$PGE_2$. When $R_1$ is $OR_{18}$, $R_2$ is n–$C_4H_9$, and $L_1$ is α–H:β–H, the process is used to prepare $PGF_{2\alpha}$.

By "borohydride salt" is meant any of the known borohydride salts which are used as reducing agents. These include particularly those formed with metal cations.

Especially preferred cations are those derived from the alkali metals, e.g. lithium, sodium and potassium, and from the alkaline earth metals, e.g. magnesium and calcium, although cationic forms of other metals as well as organic cations such as quaternary ammonium and quaternary phosphonium are within the scope of this invention. The alkali metal salts, particularly sodium borohydride, are preferred.

By "trivalent lanthanide salt" is meant a trivalent salt formed with any of the following elements: cerium, (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb) and lutetium (Lu). Suitable trivalent salts formed with these elements include the halides (e.g. chloride, bromide, fluoride, iodide), nitrates, sulfate, sulfonates and phosphates. The chlorides are preferred for use in this invention.

Cerous trichloride ($CeCl_3$) is the most preferred lanthanide salt to be used in this invention because of its relatively low cost and the favourable ratio of α/β epimers obtained. Although $SmCl_3$ produces a slightly more favourable α/β ratio, this reagent is much more expensive, making it less preferred.

$R_{18}$ includes silyl groups of the formula –Si$(G_1)_3$. $G_1$ is $C_{1-4}$ alkyl, $C_{3-10}$ cycloalkyl, $C_{7-12}$ aralkyl, phenyl or phenyl substituted by one or 2 substituents selected from F, Cl and $C_{1-4}$ alkyl. The $G_1$'s are the same or different and at least one $G_1$ is hindered (such as tert-butyl). Examples of alkyl are methyl, ethyl, isopropyl, propyl, isobutyl, butyl, sec-butyl, tert-butyl and pentyl. Examples of aralkyl are benzyl, phenethyl, α-phenylethyl, 3-phenylpropyl, α-naphthylmethyl and 2-(α-naphthyl)ethyl. Examples of substituted phenyl are p-chlorophenyl, m-fluorophenyl, o-tolyl, 2,4-dichlorophenyl, p-tert-butylphenyl, 4-chloro-2-methylphenyl and 2,4-dichloro-3-methylphenyl. Silyl groups within the scope of –Si$(G_1)_3$ include triethylsilyl, triisopropylsilyl, triphenylsilyl, t-butyldemethylsilyl, methylphenylbenzylsilyl and tert-butyldiphenyl silyl. $G_1$ may also be a phenoxy group; for example, a silyl protecting group containing a phenoxy group that has been disclosed in the literature is [2,4,6-tri(tert-butyl)-phenoxy]dimethylsilyl. Tert-butyldimethylsilyl is most preferred.

These silyl groups are known in the art. See for example, Pierce "Silylating of Organic Compounds", Pierce Chemical Company, Rockford, I11., USA (1968). When silylated products of the invention are intended to be subjected to chromatographic purification, then the use of silyl groups known to be unstable to chromatography is to be avoided.

The silyl protective groups as defined by $R_{18}$ are removed by mild acidic hydrolysis. For example, by reaction with (1) hydrochloric acid in methanol, (2) a mixture of acetic acid, water and tetrahydrofuran, or (3) aqueous citric acid or aqueous phosphoric acid in tetrahydrofuran, at temperatures below 55°C, hydrolysis of the blocking group is achieved.

According to the process of this invention, an enone compound (either optically active or a racemic mixture) of formula A-1 is treated with a borohydride salt (e.g. sodium borohydride) and a lanthanide salt (e.g. cerium trichloride) in an inert solvent at a low temperature, to yield the corresponding 15α-hydroxy compound. These enones are well known and readily available to those skilled in the art. For example, the compound of formula III is disclosed in US-A 4 052 446. Typically, the procedure employs 0.25 to 1.00 moles of borohydride salt and 0.10 to 1.00 moles of lanthanide salt per mole of enone, and more preferably about 0.60 moles of the borohydride and about 0.25 moles of the lanthanide. The temperature used for this reduction is preferably from -100 to 0 C, and more preferably –78 to –25 C, e.g. about – 78 C.

The formula A-2 product can be readily converted to corresponding prostaglandins by known means, e.g. as described by Corey et al, supra.

The following Examples illustrate the invention. Liquid proportions are by volume.

Example 1 ($R_1$ is methyl, $R_2$ is n-$C_4H_9$, $L_1$ is α-$CH_3$:β-$CH_3$)

A solution of the optically active enone (15.23 g, 52.1 mmoles) and cerous chloride heptahydrate ($CeCl_3.7H_2O$) (4.86 g, 13.03 mmoles) in 170 ml methanol and 50 ml methylene chloride is cooled to - 78 C (internal temp. -67 C), and treated with solid sodium borohydride ($NaBH_4$) (1.097 g, 29.0 mmoles). The reaction is incomplete after 40 mins by TLC, so more $NaBH_4$ (106 mg, 2.82 mmoles) is added. After another 20 min, the reaction is judged to be complete, so it is quenched by the addition of 25 ml saturated ammonium chloride ($NH_4Cl$). The reaction mixture is then allowed to warm up to room temperature, poured into 800 ml saturated $NH_4Cl$, and extracted with ethyl acetate (2 x 500 ml). The extracts are then dried and concentrated to leave 16.30 g of a pale yellow oil.

In order to determine the 15α/β ratio, a small-scale experiment (1.099 g enone) was carried out by the procedure described above. The crude product mixture was chromatographed on silica gel (gradient elution, methylene chloride to 10% acetone/methylene chloride). The fractions containing the desired 15α

isomer (Rf=0.47; eluant: 5% acetone/methylene chloride) were combined and concentrated in vacuo to leave a pale yellow oil (weight: 818 mg, 73.9%). The fractions containing the 15β isomer (Rf=0.30) were then combined and concentrated in vacuo to leave a pale yellow oil (weight: 180 mg, 16.3%). The 15α/β weight ratio is therefore 82:18.

Example 2

The effects of various reaction parameters are set forth in Tables I, II and III. In all cases, $R_1$, $R_2$ and L are as defined in Example 1, and the enone A-I is optically active.

Table I

| Reducing Agent/Temp. (°C) | 15-epi-A-1 | A-2 | A-2 | Composition of Product Mixture 15 α/β | 13,14-Dihydro-A-2 (a/β) | IV, 15-epi-IV (α/β) |
|---|---|---|---|---|---|---|
| $NaBH_4$, MeOH,/0° | trace | 50.6 | 32.3 | 61.0/39.0 | 6.1 (52/48) | 1.9 (63/37) |
| DIBAL/BHT, #Me,/–24° | trace | 50.5 | 49.5 | 50.5/49.5 | <1.0% | trace |
| $NaBH_4$, $CeCl_3$, MeOH,/–78° | .10 | 78.6 | 17.0 | 82.3/17.7 | .04 (–) | .60 (70/30) |

Table II

(Effect of Co-solvent on Product, 0.25 equiv. $CeCl_3$, –78°)

| Solvents | 15-epi-A-1 | A-2 | A-2 | 15 α/β | 13,14-Dihydro (a/β) | IV, 15-epi-IV (α/β) |
|---|---|---|---|---|---|---|
| MeOH | .10 | 78.6 | 17.0 | 82.3/17.7 | .04 (–) | .60 (70/30) |
| MeOH/THF | <.1 | 75.9 | 19.6 | 79.5/20.5 | <.1 | 4.4 (79/21) |
| MeOH/$CH_2Cl_2$ | 1.7 | 77.8 | 15.2 | 83.7/16.3 | <.1 | 1.9 (81/19) |

Table III

(Effect of Temperature on Product Distribution; .25 equiv. $CeCl_3$)

| Solvents/Temp. (°C) | 15-epi-A-1 | A-2 | A-2 | 15 α/β | 13,14-Dihydro-A-2 (a/β) | Triols (α/β) |
|---|---|---|---|---|---|---|
| MeOH, $CH_2Cl_2$/–78° | 1.7 | 77.8 | 15.2 | 83.7/16.3 | <.1 | 1.9 (81/19) |
| MeOH, $CH_2Cl_2$/–30° | 2.5 | 65.5 | 20.12 | 76.4/23.6 | <.1 | 5.2 (77/23) |
| EtOH, $CH_2Cl_2$/–20° | 3.9 | 73.1 | 19.9 | 78.6/21.4 | .7 | 3.5 (77/23) |

Example 3 Cerium Borohydride Reduction

A solution of the optically active Formula A-1 enone, wherein $R_1$ is $CH_3$, $R_2$ is $nC_4H_9$, and $L_1$ is α-$CH_3$:β-$CH_3$ (8.297 g, 28.42 mmoles), and cerous chloride heptahydrate (2.651 g, 7.115 mmoles) in 80 ml methanol and 50 ml methylene chloride is cooled to - 76° C. To the resulting cloudy solution is then added solid sodium borohydride (0.650 g, 17.113 mmoles) all at once. The reaction mixture is then stirred at -76° C until a TLC (eluant: 40% ethyl acetate/cyclohexane indicates that the amount of product (Rf=0.38) is maximal (60 min; at this point roughly equal amounts of unreacted enone (Rf=0.50) and the triol C-15 epimer pair [IV, Rf=0.10 and 15-epi-IV, Rf=0.05] are present). The reaction mixture is then quenched with 50 ml saturated aqueous ammonium chloride ($NH_4Cl$), allowed to warm to room temperature, poured into 400 ml saturated aqueous $NH_4Cl$, and extracted with ethyl acetate (2 x 200 ml). The extracts are then washed successively with 100 ml saturated aqueous $NH_4Cl$ and 100 ml saturated aq. sodium chloride, dried over magnesium sulfate, and concentrated in vacuo to leave a pale yellow oil consisting of a 83.7:15.6 mixture of the desired product A-2 ($R_1$ is $CH_3$, $R_2$ is $nC_4H_9$ and $L_1$ is a α-$CH_2$:β-$CH_3$) and its epimer (by HPLC).

The desired product weighs 8.468 g.

Example 4

The optically active enone of Formula A-1, wherein $R_1$ is $CH_3$, $R_2$ is $nC_4H_9$, and $L_1$ is $\alpha$-$CH_3$:$\beta$-$CH_3$ is treated with sodium borohydride in the presence of all but the four most expensive lanthanide trichloride hydrates (Pm, Ho, Tm, and Lu) under the conditions of Example 3. The results are set forth in Table IV.

Table IV

| Effect of Lanthanide on Product Distribution | |
|---|---|
| Lanthanide | Ratio $\alpha/\beta$ |
| None | 61.0/39.0 (1,2/1,4 = 94/6) |
| Ce | 84.3/15.7 |
| Pr | 84.3/15.7 |
| Nd | 83.0/17.0 |
| Sm | 85.4/14.6 |
| Eu | 79.6/20.4 |
| Gd | 84.0/16.0 |
| Tb | 80.5/19.5 |
| Dy | 81.4/18.6 |
| Er | 81.2/18.8 |
| Yb | 78.9/21.1 |

Example 5 A comparison of the results of reduction of optically active racemic enones A-1 ($R_1$ is SitBuMe$_2$, $R_2$ is $nC_4H_9$ and $L_1$ is $\alpha$-H:$\beta$-H) to A-2

The results are set forth in Table V

Table V

| Substrate | 15 $\alpha/\beta$ |
|---|---|
| optically active | 70.9/29.1 |
| racemic | 71.6/28.4 |

I

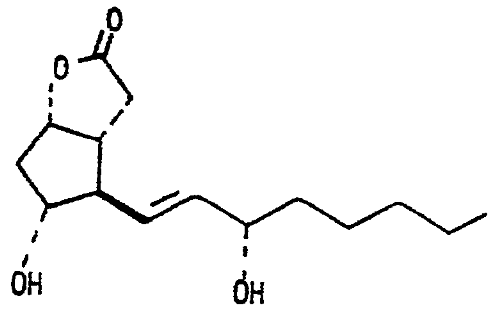

II

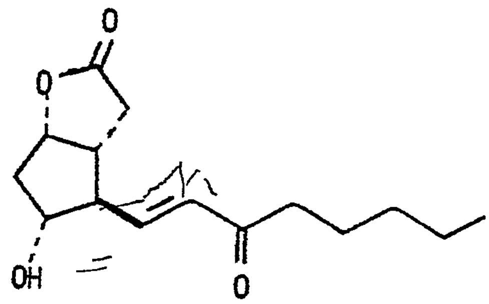

III

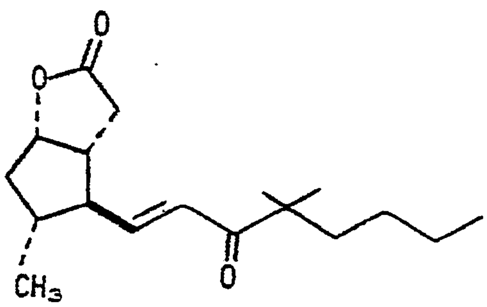

IV

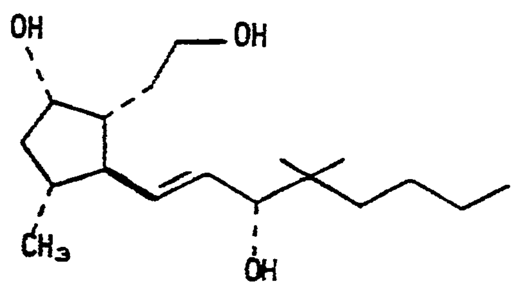

## CHART A

A-1

A-2

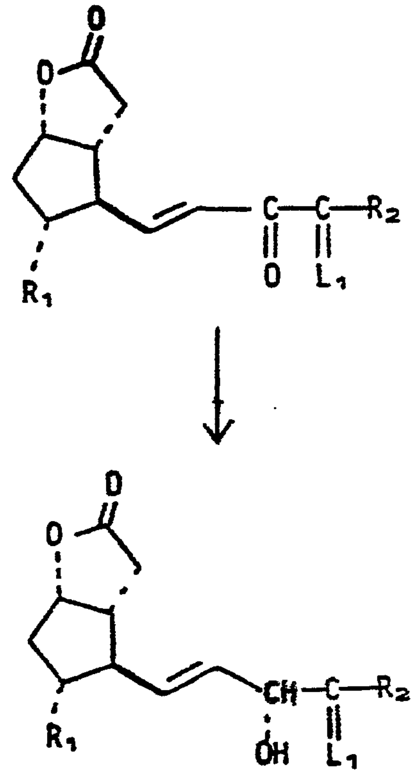

## Claims

1. A process for the stereo-specific preparation of a compound of the formula

A-2

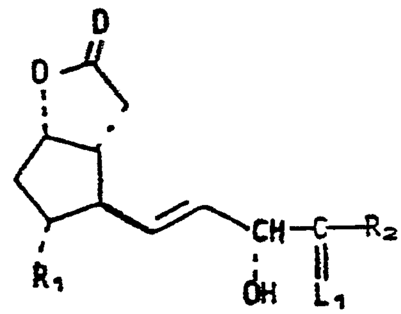

wherein $R_1$ is Methyl or $OR_{18}$, $R_{18}$ being a silyl protecting group;

$R_2$ is -O-(PhX), $-C_pH_{2p}$-(PhX), $-C_mH_{2m}$-(DZ), $-C_pH_{2p+1}$, $-CH_2-CH_2-CH=C(CH_3)_2$, 3-thienyloxy or $-C_pH_{2p}$-(Py) ; and

$L_1$ is $\alpha$-$R_9$:$\beta$-$R_{10}$, $\alpha$-$R_{10}$:$\beta$-$R_9$, $\alpha$-$OR_8$:$\beta$-$R_7$ or $\alpha$-$R_7$:$\beta$-$OR_8$;

in which (PhX) is phenyl optionally substituted by up to 3 substituents independently selected from $C_{1-4}$ alkyl, Cl, F, Br, $NO_2$, $CF_3$, OH and $C_{1-4}$ alkoxy;

(DZ) is a $C_{3-6}$ cycloaliphatic radical optionally substituted by up to 3 substituents as defined above for (PhX);

(Py) is 2, 3 or 4-pyridinyl;

$R_7$ and $R_8$ are independently selected from H and $C_{1-4}$ alkyl;

$R_9$ and $R_{10}$ are independently selected from H, $C_{1-4}$ alkyl and F;
m is zero, 1, 2 or 3; and
p is zero or an integer of from 1 to 8;
which comprises reducing a compound of the formula

A-1

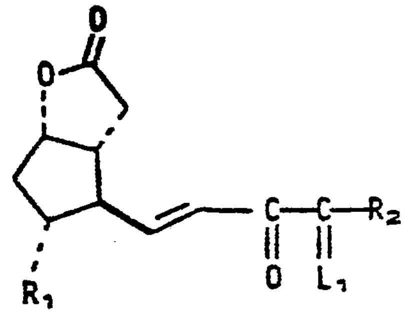

wherein $R_1$, $R_2$ and $L_1$ are as defined above, with a borohydride salt, in the presence of a lanthanide salt.

2. A process according to claim 1, wherein $R_1$ is $CH_3$, $R_2$ is n-$C_4H_9$, and $L_1$ is $\alpha$-$CH_3$:$\beta CH_3$.
3. A process according to claim 1, wherein $R_1$ is -$OR_{18}$, $R_2$ is n-$C_4H_9$, and $L_1$ is $\alpha$-H:$\beta$-H.
4. A process according to any preceding claim, wherein the borohydride salt is sodium borohydride.
5. A process according to any preceding claim, wherein the lanthanide salt is a lanthanide halide.
6. A process according to claim 5, wherein the lanthanide halide is cerous trichloride.
7. A process according to any preceding claim, which is conducted at from -100 to 0 C.

**Patentansprüche**

1. Ein Verfahren für die stereospezifische Zubereitung einer Verbindung der Formel

A-2

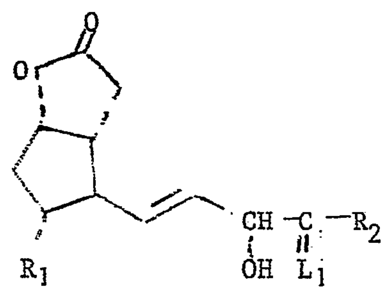

in der $R_1$ Methyl oder $OR_{18}$ ist, wobei $R_{18}$ eine Silylschutzgruppe bildet;
$R_2$ –O–(PhX), –$C_pH_{2p}$–(PhX), –$C_mH_{2m}$–(DZ), –$C_pH_{2p+1}$, –$CH_2$–$CH_2$–CH=C$(CH_3)_2$, 3-Thienyloxy oder –$C_pH_{2p}$–(Py) ist; und
$L_1$ $\alpha$–$R_9$:$\beta$–$R_{10}$, $\alpha$–$R_{10}$:$\beta$–$R_9$, $\alpha$–$OR_8$:$\beta$–$R_7$ oder $\alpha$–$R_7$:$\beta$–$OR_8$ ist;
worin (PhX) Phenyl ist, das wahlweise durch bis 3 unabhängig aus der Gruppe $C_{1-4}$ Alkyl, Cl, F, Br, $NO_2$, $CF_3$, OH und $C_{1-4}$ Alkoxy ausgewählten Substituenten substituiert ist;
(DZ) ein $C_{3-6}$ cycloaliphatisches Radikal ist, das wahlweise durch bis 3 wie vorstehend für (PhX) definierte Substituenten substituiert ist;
(Py) 2, 3 oder 4-Pyridinyl ist;
$R_7$ und $R_8$ unabhängig aus der Gruppe H und $C_{1-4}$ Alkyl ausgewählt sind;
$R_9$ und $R_{10}$ unabhängig aus der Gruppe H, $C_{1-4}$ Alkyl und F ausgewählt sind;
m Null, 1, 2 oder 3 ist; und
p Null oder eine ganze Zahl von 1 bis 8 ist;
wobei das besagte Verfahren sich auf Reduktion einer Verbindung der Formel

A-1

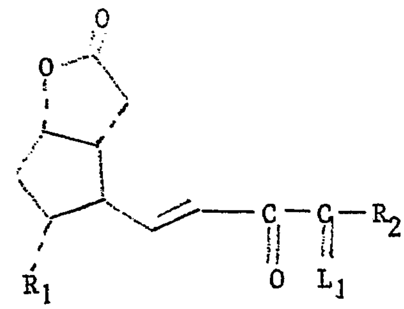

erstreckt, in der $R_1$, $R_2$ und $L_1$ wie vorstehend definiert sind, und zwar durch ein Borhydridsalz in Gegenwart eines Lanthanidsalzes.

2. Ein Verfahren nach Anspruch 1, in dem $R_1$ $CH_3$ ist, $R_2$ n–$C_4H_9$ ist und $L_1$ α–$CH_3$:β–$CH_3$ ist.

3. Ein Verfahren nach Anspruch 1, in dem $R_1$ –$OR_{18}$ ist, $R_2$ n–$C_4H_9$ ist und $L_1$ α–H:β–H ist.

4. Ein Verfahren nach einem der vorstehenden Ansprüche, bei dem das Borhydridsalz Natriumborhydrid ist.

5. Ein Verfahren nach einem der vorstehenden Ansprüche, bei dem das Lanthanidsalz ein Lanthanidhalid ist.

6. Ein Verfahren nach Anspruch 5, bei dem das Lanthanidhalid Cerotrichlorid ist.

7. Ein Verfahren nach einem der vorstehenden Ansprüche, das bei Temperaturen von –100 bis 0°C durchgeführt wird.

**Revendications**

1. Procédé de préparation stéréospécifique d'un composé de formule

A-2

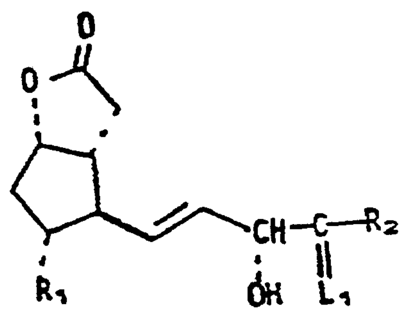

dans laquelle $R_1$ représente un groupe méthyle ou $OR_{18}$, $R_{18}$ étant un groupe protecteur silyle;
$R_2$ représente un groupe –O–(PhX), –$C_pH_{2p}$–(PhX), –$C_mH_{2m}$–(DZ), –$C_pH_{2p+1}$, –$CH_2$–$CH_2$–$CH$=$C(CH_3)_2$, 3-thiényloxy ou –$C_pH_{2p}$–(Py); et
$L_1$ représente un groupe α–$R_9$:β–$R_{10}$, α–$R_{10}$:β–$R_9$, α–$OR_8$:β–$R_7$ ou α–$R_7$:β–$OR_8$;
dans lesquels (PhX) représente un groupe phényle facultativement substitué avec jusqu'à 3 substituants choisis indépendamment entre des groupes alkyle en $C_1$ à $C_4$, Cl, F, Br, $NO_2$, $CF_3$, OH et alkoxy en $C_1$ à $C_4$;
(DZ) représente un radical cycloaliphatique en $C_3$ à $C_6$ substitué facultativement avec jusqu'à 3 substituants répondant à la définition précitée pour (PhX);
(Py) représente un groupe 2-, 3- ou 4-pyridinyle;
$R_7$ et $R_8$ sont choisis indépendamment entre H et un groupe alkyle en $C_1$ à $C_4$;
$R_9$ et $R_{10}$ sont choisis indépendamment entre H, un groupe alkyle en $C_1$ à $C_4$ et F;
m a la valeur zéro, 1, 2 ou 3; et
p est égal à zéro ou à un nombre entier de 1 à 8;
qui consiste à réduire un composé de formule

A-1

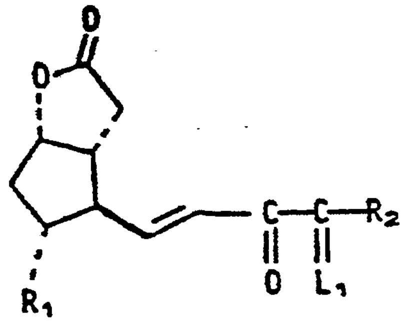

dans laquelle $R_1$, $R_2$ et $L_1$ répondent aux définitions précitées, avec un borohydrure, en présence d'un sel de lanthanide.

2. Procédé suivant la revendication 1, dans lequel $R_1$ représente un groupe $CH_3$, $R_2$ représente un groupe $n-C_4H_9$ et $L_1$ représente un groupe $\alpha-CH_3:\beta-CH_3$.

3. Procédé suivant la revendication 1, dans lequel $R_1$ représente un groupe $-OR_{18}$, $R_2$ représente un groupe $n-C_4H_9$ et $L_1$ représente un groupe $\alpha-H:\beta-H$.

4. Procédé suivant l'une quelconque des revendications précédentes, dans lequel le borohydrure est le borohydrure de sodium.

5. Procédé suivant l'une quelconque des revendications précédentes, dans lequel le sel de lanthanide est un halogénure de lanthanide.

6. Procédé suivant la revendication 5, dans lequel l'halogénure de lanthanide est le trichlorure céreux.

7. Procédé suivant l'une quelconque des revendications précédentes, qui est mis en œuvre à une température de –100 à 0°C.